# EUROPÄISCHE PATENTSCHRIFT

(11) **EP 0 496 696 B1**
(45) Veröffentlichungstag und Bekanntmachung des Hinweises auf die Patenterteilung: **30.11.1994**
(21) Anmeldenummer: 92710003.2
(22) Anmeldetag: 21.01.1992
(51) Int. Cl.: A41D 13/12

(54) **Bekleidungsstück zur Verwendung bei juckenden Hautkrankheiten**
Cloth for skin itching disease
Vêtement à utiliser lors de maladies prurigineuses

(30) Priorität: 21.01.1991 DE 4101580
(43) Veröffentlichungstag der Anmeldung: 29.07.1992
(73) Patentinhaber: Lohmann GmbH & Co. KG, 56567 Neuwied (DE)
(72) Erfinder: Fäth, Martina, D-63857 Waldaschaff (DE)
(74) Vertreter: Harders, Gerhard, Dr.

(56) Entgegenhaltungen:
- CA-A- 916 352
- DE-U- 8 703 376
- FR-A- 2 168 889
- US-A- 2 008 152
- US-A- 2 291 861
- US-A- 2 621 336

## Beschreibung

Die vorliegende Erfindung betrifft ein Bekleidungsstück zur Verwendung bei juckenden Hautkrankheiten.

Bei juckenden Hautkrankheiten, wie zum Beispiel Windpocken, Masern,. Hautallergien, insbesondere bei Neurodermitis, stellt sich häufig ein starker Juckreiz ein, der sich bei dem betroffenen Menschen vom Kopf bis zur Sohle erstrecken kann und hauptsächlich an den Halspartien, Armbeugen und Kniegelenken auftreten kann. Insbesondere bei Neurodermitis, deren Ursachen in Umwelteinflüssen, Streß, der Ernährung oder psychischen Einflüssen liegen kann, sammelt sich unter der Hautoberschicht eine Flüssigkeit an, die einen außergewöhnlich starken Juckreiz hervorruft. Durch das dadurch verursachte Kratzen läuft zwar die angesammelte Flüssigkeit ab, es entstehen aber tiefe Wunden und Hautrisse, die sich flach den heutigen Erkenntnissen medikamentös nur schwierig beeinflussen lassen. Bei durch Neurodermitis hervorgerufenen Allergieschüben kann der Juckreiz ein bis zwei Wochen anhalten, wodurch die Haut bis zum Bluten gekratzt wird und eine nicht unbeträchtliche Infektionsgefahr besteht.

Ein gattungsgemäßes Bekleidungsstück gemäß den Merkmalen des Oberbegriffs des Hauptanspruchs ist in der US-A-2 008 152 beschrieben. Es enthält keinen eng verstellbaren Armabschluß, die Verwendung bei juckenden Hautkrankheiten ist nicht angesprochen.

Aufgabe der vorliegenden Erfindung ist es, ein Bekleidungsstück zu schaffen, das es ermöglicht, den bei vorgenannten Krankheiten auftretenden Kratzdrang so abzufangen, daß die oben erwähnten Nachteile behoben werden, und bei dem ein Herausschlüpfen der Hände aus den Armteilen vermieden wird, sodaß die das Bekleidungsstück tragende Person sich nicht innerhalb des Bekleidungsstücks kratzen kann.

Gelöst wird diese Aufgabe durch ein Bekleidungsstück gemäß dem Kennzeichen des Hauptanspruches. Die Unteransprüche betreffen dabei bevorzugte Ausgestaltungen der Erfindung.

Durch die Ausgestaltung des Schlafoveralls mit völlig geschlossenen Armteilen und völlig geschlossenen Beinteilen wird verhindert, daß die Haut blutig gekratzt werden kann. Die Hände, insbesondere die Fingernägel sind durch den Stoff der Armteile, insbesondere der Fäustlinge geschützt, dadurch können die betroffenen Menschen ihren Kratzdrang ausüben, wobei durch den Stoffschutz nur die Hautoberschicht mechanisch gereizt wird, so daß die angesammelte Flüssigkeit abfließen kann. Der gesamte Körper steckt in einer Schutzhülle aus Textilstoff, er ist damit gegen die Kratzattacken geschützt. Als Textilstoff werden hautverträgliche Stoffe verwendet, insbesondere Textilien aus Naturfasern, wobei Baumwolle besonders bevorzugt ist, da sie von den betroffenen Menschen gut vertragen wird, eine große Saugfähigkeit hat und atmungsaktiv ist, wodurch Hitzestaus am Körper vermieden werden.

Auf diese Weise werden schwere Infektionen der Haut durch die Schutzhülle aus Naturfasern vermindert bzw. verhindert. Dies ist insbesondere für Kinder wichtig, die mit dem erfindungsgemäßen Bekleidungsstück besser schlafen, da der Körper gut geschützt ist, sodaß die Haut nicht mehr blutig gekratzt wird. Dies hat auch einen positiven Einfluß auf die Psyche des Kindes, die bei der Benutzung des Schlafoveralls nicht mehr so stark belastet wird, da das Kind sich nicht mehr blutig kratzen kann.

Da die Armteile und die Beinteile des Bekleidungsstückes völlig geschlossen sind, besteht keine Möglichkeit mehr, daß die Hände aus dem Bekleidungsstück heraus ins Freie gelangen oder innerhalb des Bekleidungsstückes juckende Körperteile kratzen können. Dabei ist es besonders bevorzugt, daß die Armteile vom Armabschluß an nach Art eines Fäustlings gearbeitet sind, die die Hände völlig bedecken. Werden die Fäustlinge aus einem verstärkten Stoff gearbeitet, so lassen sich die betroffenen Körperteile gegen Kratzattacken noch besser schützen. Dabei kann der Armabschluß, d.h. die Verbindung zwischen Armteil und Fäustling, aus einem weichen, atmungsaktiven verstellbaren Lochgummiband bestehen, das mit einem Knopf so eng geknöpft werden kann, daß die Hände nicht mehr aus dem Armteil herausschlüpfen können.

Um das Bekleidungsstück eng anliegend an den Körper auszugestalten, ist es bevorzugt, zum Verschließen des Bekleidungsstückes einen oder mehrere Reißverschlüsse vorzusehen, die vorzugsweise an der Innenseite verdeckt gearbeitet sind, um eine unmittelbare Berührung des Reißverschlusses mit dem Körper zu vermeiden. Zusätzlich kann auch das Körperstück in seinem Halsteil mit einem Halsabschluß versehen sein, der durch ein Stoffband betätigt werden kann. An den Halsabschluß anschließend kann eine Kapuze vorgesehen sein, die den Kopf bis auf das Gesicht bedeckt. Auch diese Kapuze kann durch ein Stoffband eng anliegend an den Kopf geschlossen werden. Weiterhin kann der Gürtelregion des Körperstücks ein Stoffgürtel vorgesehen sein, um auch diese zusätzlich abzuschließen. Der Stoffgürtel kann zusätzlich einen Gummizug aufweisen.

Die Erfindung wird nachstehend unter Bezugnahme auf die Figuren beispielhaft beschrieben.
- Figur 1 zeigt: einen Schlafoverall in Gesamtansicht und

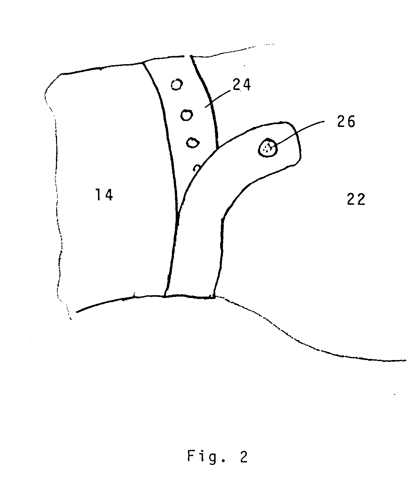

- Figur 2 zeigt: eine Detaillansicht des Armabschlusses.

Der Schlafoverall 10 weist ein Körperstück 12 auf, das mit angenähten geschlossenen Armteilen 14 und geschlossenen Beinteilen 16 gefertigt ist. Im Halsteil 30 ist ein Halsabschluß 32 vorgesehen, der durch ein Stoffband betätigt wird. Anschließend an den Halsabschluß 32 ist eine Kapuze 38 angesetzt, die den Kopf bis auf das Gesicht bedecken kann.

Zum Schutz der Hände sind die Armteile 14 mit Armabschlüssen 20 versehen, an die Fäustlinge 22 angesetzt sind. Die Fäustlinge 22 sind aus einer stabileren Stoffqualität gearbeitet, sie sind mit einem weichen, atmungsaktiven verstellbaren Lochgummiband 24 versehen, das mit einem Spezialknopf26 mit der gewünschten Spannung geknöpft werden kann.

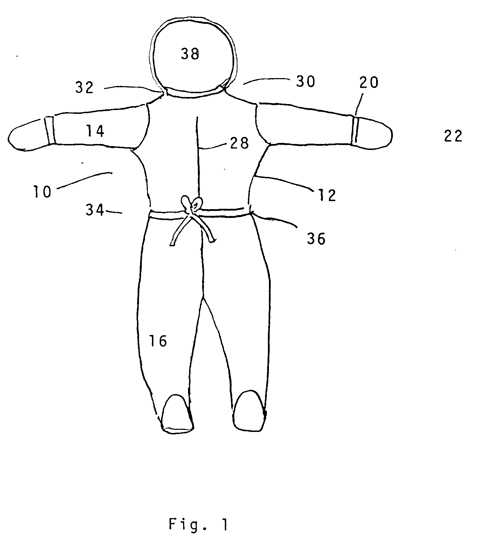

Der Schlafoverall ist mit einem Reißverschluß 28 verschließbar, der an der Innenseite verdeckt gearbeitet ist, so daß er nicht mit der nackten Haut in Berührung kommt. Bei kleinen Größen des Schlafoveralls ist es zweckmäßig, den Reißverschluß wie in Figur 1 gezeigt an der Vorderseite anzuordnen, wobei dieser bis zur Rückentaille geführt werden kann, damit die Windeln besser gewechselt werden können. Bei den Größen für größere Kinder bzw. für Erwachsene wird der Reißverschluß vorzugsweise im Rücken des Schlafoveralls angeordnet.

Der Halsabschluß 32 kann durch ein eingezogenes Stoffband zusätzlich verschlossen werden, ebenso kann die Taille in der Gürtelregion 34 durch einen Stoffgürtel 36 zusätzlich verschlossen werden, wobei der Stoffgürtel 36 auch einen Gummizug aufweisen kann.

Als Sonderaustattung sind auch Fausthandschuhe vorgesehen, die man bei einem Allergieschub zusätzlich über die Fäustlinge 22 ziehen kann, um eine weitere Milderung der Kratzwirkung zu erzielen.

Durch die völlig geschlossene Anordnung der Beinteile 16 und insbesondere der Armteile 14 wird erreicht, daß der betreffende Mensch juckende Körperteile nicht mehr mit den Fuß-, bzw. den Fingernägeln kratzen kann, vielmehr wird der nicht zu unterdrückende Kratzdrang durch den Stoffschutz soweit abgemildert, daß die Hautoberschicht zwar noch mechanisch gereitzt wird, aber nicht mehr in größerem Umfang geschädigt wird, wodurch eine beträchtliche Linderung der Beschwerden bei juckenden Hautkrankheiten, insbesondere bei Neurodermitis erreicht wird.

Die Armbeugen und Kniekehlen bestehen vorzugsweise aus einem verstärkten Stoff.

## Patentansprüche

1. Bekleidungsstück in Form eines Schlafoveralls (10) aus einem Körperstück (12) mit völlig geschlossenen Armteilen (14) und völlig geschlossenen Beinteilen (16), die aus einem hautverträglichen Textilstoff gearbeitet sind, wobei die Armteile (14) nach Art eines Fäustlings (22) gearbeitete sind, die die Hände völlig bedecken,
**dadurch gekennzeichnet, daß**
zur Verwendung bei juckenden Hautkrankheiten die Armteile (14) mit Armabschlüssen (20) versehen sind, an die die Fäustlinge (22) angesetzt sind, und die zur Verhinderung eines Herausschlüpfens der Hände eng verstellbar sind.

2. Bekleidungsstück nach Anspruch 1,
**dadurch gekennzeichnet, daß**
der Armabschluß (20) aus einem weichen, atmungsaktiven verstellbaren Lochgummiband (24) besteht, das mit einem Knopf (26) eng geknöpft werden kann.

## Claims

1. Article of clothing in the form of a coverall-type sleeping suit (10) consisting of a body part (12) having completely closed sleeve parts (14) and completely closed leg parts (16), which are made of a textile material compatible with the skin, wherein said sleeve parts (14) are made in the manner of mittens (22) covering the hands completely,
**characterised in that**
for use in the case of itching skin diseases said sleeve parts (14) are provided with sleeve seams (20) which said mittens (22) are attached to and which are adjustable so as to fit closely for preventing the hands from slipping out.

2. Article of clothing according to Claim 1,
**characterised in that**
said sleeve seam (20) consists of a soft breathing adjustable punched rubber band (24) adapted to be buttoned tightly by means of a button (26).

## Revendications

1. Vêtement sous forme d'un pyjama (10) du genre d'une salopette, constitué par une partie de corps (12) aux parties de bras (14) complètement fermées, et aux parties de jambe (16) complètement fermées, qui sont faites d'une étoffe textile compatible avec la peau, dans lequel lesdites parties de manche (14) sont faites de façon de moufles (22) qui couvrent les mains complètement,
**caractérisé en ce que**
pour l'utilisation au cas de maladies prurigineuses de la peau, lesdites parties de manche (14) sont prévues des bords de manche (20) auxquels lesdites moufles (22) sont fixées, et qui sont ajustables à étroitement serrer les mains afin d'éviter que les dernières puissent en sortir.

2. Vêtement selon la revendication 1,
**caractérisé en ce que**
ledit bord de manche (20) consiste en un ruban élastique ajustable moelleux respirant poinçonné (24), qui est apte à se boutonner étroitement moyennant un bouton (26).
